# EUROPEAN PATENT SPECIFICATION

(11) **EP 3 384 780 B1**
(45) Date of publication and mention of the grant of the patent: **06.12.2023**
(21) Application number: 18165328.8
(22) Date of filing: 30.03.2018
(51) Int. Cl.: A23D 7/00, A23D 7/005, A23D 7/01, A61K 9/107

(54) **VEGETABLE OIL COMPOSITION AND USES THEREOF**
PFLANZENÖLZUSAMMENSETZUNG UND VERWENDUNGEN DAVON
COMPOSITION D'HUILE VÉGÉTALE ET SES UTILISATIONS

(30) Priority: 04.04.2017 IT 201700036744
(43) Date of publication of application: 10.10.2018
(73) Proprietor: Noivita S.R.L.S., 28100 Novara (NO) (IT)
(72) Inventor: UBERTI, Francesca, 28838 STRESA (VERBANO CUSIO OSSOLA) (IT); MOLINARI, Claudio Giuseeppe, 28100 NOVARA (IT)
(74) Representative: Biggi, Cristina

(56) References cited:
- DE-U1-202011 003 072
- US-A1- 2013 184 354
- Anon.: "Making Effective Cannabis Oil Starting Right -Organic Production Mixing Strains to Increase Cannabinoid Profiles Safe, Full Extraction Dosing Traditional Methods of Application Water Solubility and Bio-Availability - The Cannabis Budwig Protocol Avoiding Anxiety and Panic Current Studies", , 1 January 2014 (2014-01-01), pages 1-31, XP055423550, Retrieved from the Internet: URL:http://gro4me.com/NewUserManualRev10-1 3.pdf [retrieved on 2017-11-10]
- JUNTAO YIN ET AL: "Biocompatible nanoemulsions based on hemp oil and less surfactants for oral delivery of baicalein with enhanced bioavailability", INTERNATIONAL JOURNAL OF NANOMEDICINE, vol. Volume 12, 1 April 2017 (2017-04-01), pages 2923-2931, XP055422660, DOI: 10.2147/IJN.S131167

## Description

The present invention relates to a composition consisting of linseed oil and hempseed oil, characterized by a balanced omega-3 and omega-6 ratio and a volume/volume ratio between linseed oil and hempseed oil comprised between 60:40 and 95:5.

The invention also relates to a process for obtaining a nanoemulsion from the composition of linseed oil and hempseed oil and the nanoemulsion thus obtained.

Finally, a further aspect of the invention relates to the use of said composition and/or nanoemulsion as a medicament for curative/ preventive purposes or as a cosmetic.

### PRIOR ART

The term "bioavailability" is prevalently used in pharmacology and refers to one of the main pharmacokinetic properties. In particular, this term indicates the fraction of a drug that reaches systemic circulation without undergoing modifications, relative to the total amount of drug administered. By analogy with the administration of the drugs, the term bioavailability is often also used with reference to substances of dietary origin.

One of the mechanisms for increasing the bioavailability of substances useful for the body and taken orally is to increase the intestinal absorption thereof. For this purpose, different systems can be used, such as, for example, the use of organic solvents, the micronization of the substances themselves or the association of the substances with molecules having a similar biological profile.

Also fitting into this context is the use of carriers of natural origin that favour the intestinal passage of the substances of interest by virtue of their intrinsic biological properties.

At present, various refined vegetable oils are commonly used as carriers of natural origin to increase the intestinal absorption of pharmaceutical preparations and food supplements.

A publication of the Stoney girl gardens of 2014, entitled "Making effective cannabis oil starting right" discloses a mixture of cannabis oil and linseed oil in various amounts. The cannabis oil is extracted from the whole plant except the stem. In the extraction process the whole plant is extracted in alcohol at a temperature of 190°F, about 87°-88°C, for 3h. The extract is subsequently filtered, and the alcohol is distilled. Different types of hemp are used for the extraction. The obtained extract contains a high quantity of cannabinoids and an unspecified amount of saturated and unsaturated fatty acids.

A publication of Juntao Yin et al, of 2014, entitled "biocompatible nanoemulsion based on hemp oil and less surfactant for oral delivery of baicalein with enhanced bioavailability" discloses the production of nanoemulsions containing the anti-inflammatory flavonoid baicalein as well as hemp oil (ratio of omega-6 to omega-3 = 2-3:1). The composition is used orally as a medicament.

However, such oils have several disadvantages, prevalently correlated with the refinement process: due to this process, in fact, the oils themselves undergo oxidation and the finished product contains undesirable chemical residues. Refined vegetable oils may thus have undergone a rearrangement or loss of the double bonds of the fatty acids contained in them. Consequently, the use of oils thus modified as carriers for substances to be orally administered may cause a loss of the membrane organization of the cells involved in intestinal absorption, with a consequent loss of fluidity and permeability of the membrane itself. The rearrangement and/or loss of the double bonds can also influence the organization and conformation of many proteins, causing different degenerative pathological conditions defined as "misfolding" diseases, in particular neurodegenerative diseases (for example Alzheimer's disease, Huntington's chorea, Parkinson's disease, amyloidosis and prion diseases), haematological diseases (for example sickle-cell anaemia), cystic fibrosis and tumours.

The use of refined vegetable oils can moreover alter the balance in the ratio between omega-6 fatty acids and omega-3 fatty acids, which is relevant from a nutritional standpoint and from the standpoint of the body's health.

Omega-6 fatty acids (or ω-6, or n-6) are a family of polyunsaturated fatty acids of vegetable origin characterized by the position of the first double bond C=C which, counting the positions starting from the terminal carbon (carbon ω carbon n), occupies the sixth position. Similarly, omega-3 fatty acids (or ω-3, or n-3) have the first double bond C=C in the third position, again counting starting from the terminal carbon.

The correct ratio between fatty acids provides that for every 4 units of ω-6, (at least) 1 of ω-3 is taken in; furthermore, the ω-6:ω-3 ratio should always be maintained below 10:1.

The ω-6:ω-3 ratio represents a criterion of nutritional balance, and several studies have demonstrated a correlation between the pathogenesis of some diseases and an alteration in the ratio between the two families of fatty acids.

Furthermore, it is known that an abuse of one or the other family of fatty acids can cause pathologies tied to an excess of oxidative stress for the body or systemic phlogosis. A correct ratio between ω-6 and ω-3 is also important for maintaining the homeostasis of blood fat and of endogenous eicosanoids, as well as for regulating arterial pressure.

In the light of the foregoing, it thus appears evident that the present use of refined vegetable oils as carriers to improve the absorption of substances useful to the body results in numerous disadvantages.

The main task of the present invention is therefore to overcome the limits of the compositions for increasing the intestinal absorption of the known substances with low bioavailability presently used in the prior art, in particular the compositions based on refined vegetable oils. Within the scope of this task, one object of the invention is to provide a carrier composition for substances with low bioavailability that favours the intestinal absorption of such substances.

Another object of the invention is to provide a carrier composition as described above which is not harmful when administered to the body. In particular, one object of the invention is to provide a composition which, in addition to having the carrier function described above, itself performs a beneficial activity, by providing an appropriate and balanced input of nutrients.

In this context, the invention proposed here has the aim of providing a carrier composition for substances with low bioavailability that is suitable for the human diet and safe for the consumer who takes it.

The last, but not least, object of the invention proposed here is to provide a composition with the above-described characteristics that is easy to produce and entails low costs.

### SUMMARY OF THE INVENTION

A first aspect of the present invention relates to a mixture consisting of two oils of vegetable origin characterized by an omega-6:omega-3 fatty acids ratio (ω6:ω3) comprised between 1:4 and 3:1; the two oils are linseed oil and hempseed oil in a volume/volume ratio between linseed oil and hempseed oil comprised between 60:40 and 95:5, preferably between 65:35 and 85:15, more preferably between 70:30 and 85:15, even more preferably between 70:30 and 80:20 and even more preferably it is 75:25. A further aspect of the present invention relates to a composition comprising said mixture and preferably at least one molecule, compound, substance characterized by: 1) low bioavailability; and/or 2) rapid metabolism; and/or 3) low water solubility, more preferably said molecule, compound, substance being selected from the group consisting of: vitamins, polyphenols, flavonoids, isoflavonoids, bioflavonoids, phytoestrogens, carotenoids, vegetable extracts, lactic ferments, melatonin, coenzyme Q10, lipophilic substances, molecules with a particularly rapid metabolism and combinations thereof. The vitamins are preferably selected from the group consisting of: vitamin A, vitamin C, vitamin D, preferably vitamin D3, vitamin E, vitamin K and combinations thereof; 2) the polyphenols are selected from the group consisting of: anthocyanins, curcumin, resveratrol, silimarin and combinations thereof; 3) the phytoestrogens are preferably isoflavones, more preferably selected from the group consisting of: genistein, daidzin, glycitein, cranberry and combinations thereof; 4) the carotenoids are selected from the group consisting of: astaxanthin, beta-carotene, crocetin, lycopene, lutein, zeaxanthin and combinations thereof; 5) the preferred flavonoid is quercetin.

A further aspect of the present invention relates to a process for nanoemulsifying the above-described mixture comprising at least one step, preferably 3-10 mixing steps, at a temperature lower than 10°C, wherein said mixing step is preferably carried out in rotation for about 1-10 seconds, preferably 3-5 seconds, wherein said rotation is preferably carried out at 8000-16000 rpm (revolutions per minute), preferably at 9000-11000 rpm, more preferably at 10000 rpm, said rotation being preferably carried out by means of an emulsifier.

Therefore, a further aspect of the present invention relates to a nanoemulsion obtained/obtainable with the process described above.

A further aspect of the present invention relates to the mixture and/or the composition and/or the nanoemulsion formulated as lozenges, tablets, pills, granules, preferably for oral administration, or as a cream, gel, lotion, ointment, spray, tissue, preferably for topical applications, preferably for sports and/or cosmetic and/or nutraceutical applications.

A further aspect of the present invention relates to the use of the mixture and/or of the composition and/or of the nanoemulsion as a cosmetic. A further aspect of the present invention relates to the use of the mixture and/or of the composition and/or of the nanoemulsion to increase the absorption and/or the metabolism and/or the bioavailability, preferably at the intestinal site, of molecules with low bioavailability that are drugs.

A further aspect of the present invention relates to the mixture and/or the composition and/or the nanoemulsion for use as a medicament, preferably for use in the prevention and/or in the therapeutic treatment and/or in the follow-up of a pathology selected from the group consisting of degenerative diseases in the neurological, ophthalmic, gynecological, cardiovascular, gastrointestinal, osteoarticular and muscular fields and malabsorption syndrome, preferably said pathology being selected from the group consisting of: glaucoma, retinopathies and maculopathy, chronic stroke, neurodegenerative diseases, pre-menstrual syndrome and menopause, polycystic ovary syndrome, coronary heart disease, hypertension, cardiac failure, as a cardiac adjuvant for long-term pharmacological treatments, ulcer, hyperacidity and gastro-esophageal reflux, chronic inflammatory diseases (rectocolitis and Chron's disease), rheumatic diseases, and for post-traumatic and rehabilitation treatments.

### DESCRIPTION OF THE FIGURES

The present invention will be described in detail below and will be illustrated, for non-limiting demonstrative purposes, also with the aid of the following figures.

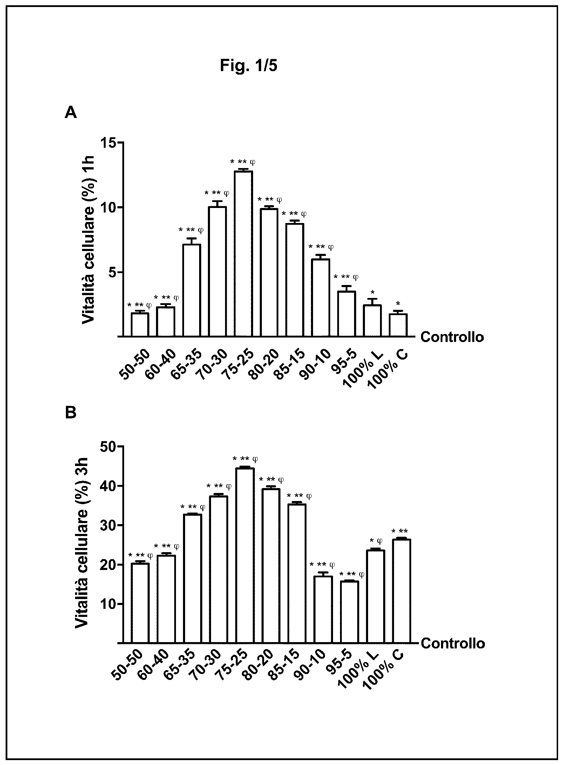

- Figure 1 shows the viability of CaCo-2 cells following treatment, after 1 hour (A) and 3 hours (B) of treatment, with examples of the composition of the invention measured by means of an MTT assay.
   L=linseed oil; C=hempseed oil. The examples of the composition are: 100%L=100% linseed oil; 100%C=100% hempseed oil; 50-50=50%L+50%C; 60-40=60%L+40%C; 65-35=65%L+35%C; 70-30=70%L+30%C; 75-25=75%L+25%C; 80-20=80%L+20%C; 85-15=85%L+15%C; 90-10=90%L+10%C; and 95-5=95%L+5%C.
   The data are expressed as the mean ± standard deviation (SD, %) normalized relative to the untreated sample (control) of 4 independent experiments, each conducted in triplicate.
   * p<0.05 vs control; ** p<0.05 vs 100%L; ϕ p<0.05 vs 100%C.

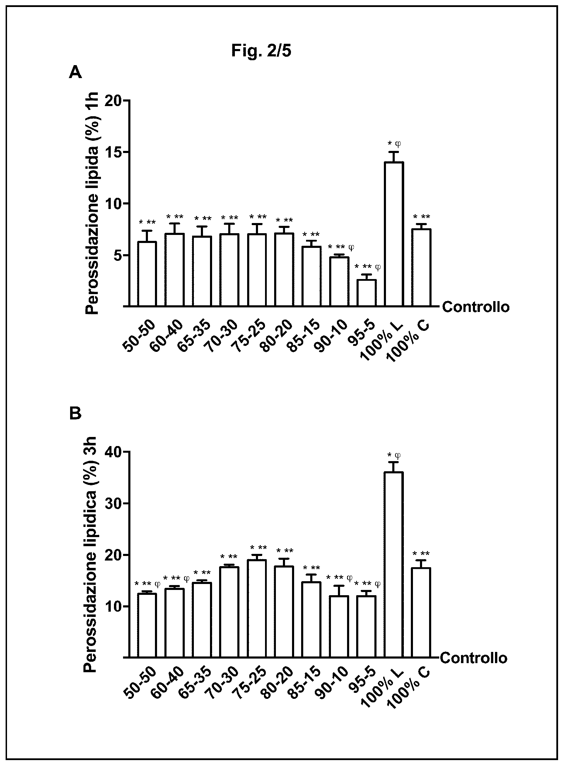

- Figure 2 shows the level of lipid peroxidation in the CaCo-2 cells after 1 hour (A) and after 3 hours (B) of treatment with examples of the composition of the invention measured by means of MDA kit.
   The tested examples of the composition are the same as in Fig.1.
   In this case as well, the data are expressed as the mean ± standard deviation (SD, %), normalized relative to the untreated sample (control) of 4 independent experiments, each conducted in triplicate.
   * p<0.05 vs control; ** p<0.05 vs 100%L; ϕ p<0.05 vs 100%C.

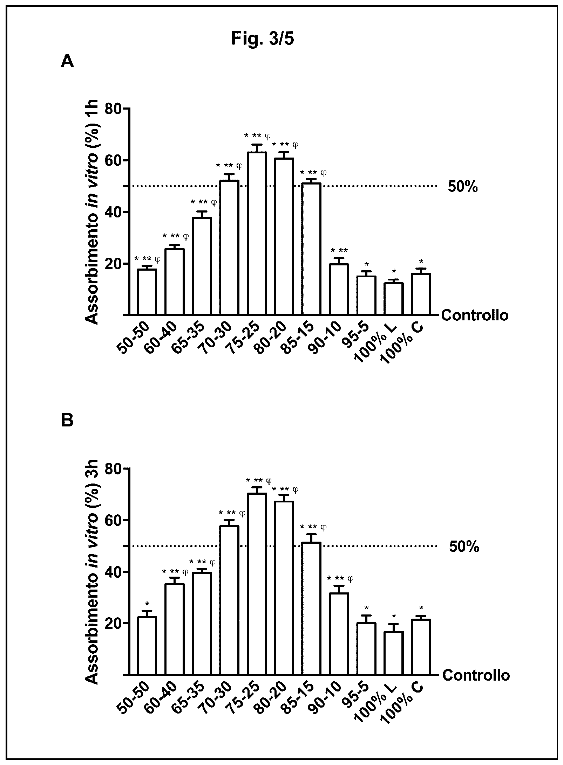

- Figure 3 shows the data of CaCo-2 cell-mediated absorption after 1 hour (A) and after 3 hours (B) of treatment with examples of the composition of the invention measured by means of a gradient.
   The tested examples of the composition are the same as in Fig.1.
   The data are expressed as the mean ± standard deviation (SD, %), normalized relative to the untreated sample (control) of 4 independent experiments, each conducted in triplicate.
   * p<0.05 vs control; ** p<0.05 vs 100%L; ϕ p<0.05 vs 100%C.

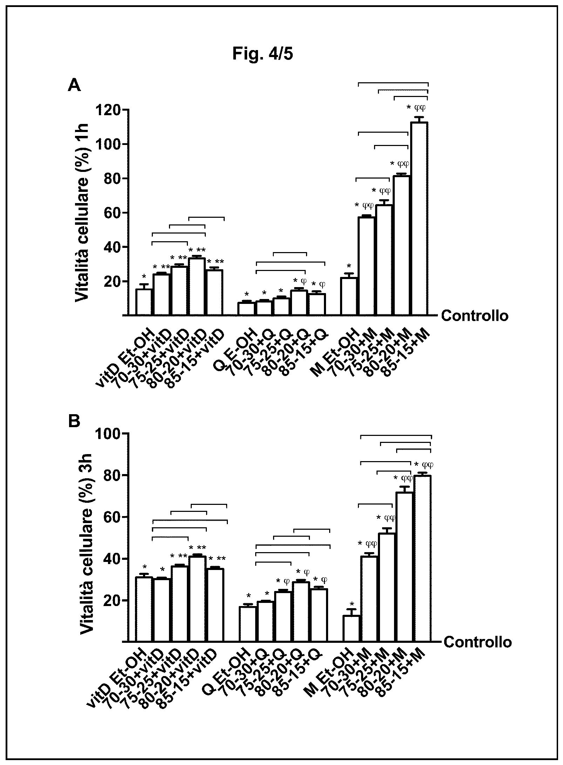

- Figure 4 shows the viability of CaCo-2 cells after 1 hour (A) and after 3 hours (B) of treatment with examples of the composition of the invention comprising, in addition to oils, vitamin D3, or vegetable oils and quercetin, or vegetable oils and melatonin, measured by means of an MTT assay.
   vitD= vitamin D3; Q= quercetin; M= melatonin.
   Vitamin D3, quercetin and melatonin are in the 70-30, 75-25, 80-20 or 85-15 compositions of linseed oil and hempseed oil (as defined in figures 1-3) or in 100% ethanol (Et-OH) at concentrations, respectively, of 100 nM, 50 µM and 50 µM.
   In this case as well, the data are expressed as the mean ± standard deviation (SD, %), normalized relative to the untreated sample (control) of 4 independent experiments, each conducted in triplicate.
   * p<0.05 vs control; ** p<0.05 vs vitD Et-OH; ϕ p<0.05 vs Q Et-OH; ϕϕ p<0.05 vs M Et-OH; the bars indicate p<0.05 vs the compositions of linseed oil and hempseed oil.

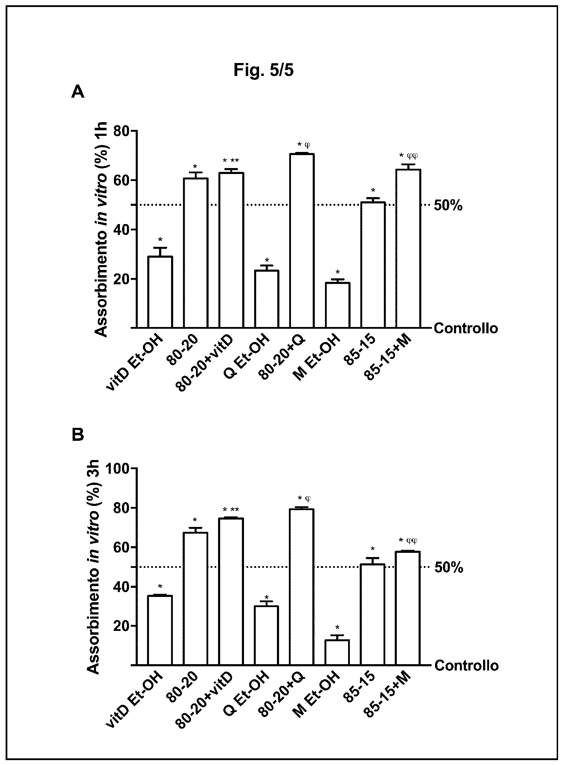

- Figure 5 shows the data of CaCo-2 cell-mediated absorption after 1 hour (A) and after 3 hours (B) of treatment with examples of the composition of the invention, in particular, 1) a composition comprising 80%L+20%C and vitamin D3 or quercetin, and 2) a composition comprising 85%L+15%C and melatonin, measured by means of a gradient.

The abbreviations are the same as in the previous figures.

The data are expressed as the mean ± standard deviation (SD, %) normalized relative to the untreated sample (control) of 4 independent experiments, each conducted in triplicate.
* p<0.05 vs control; ** p<0.05 vs vitD Et-OH; ϕ p<0.05 vs Q Et-OH; ϕϕ p<0.05 vs M Et-OH.

### DETAILED DESCRIPTION OF THE INVENTION

In the context of the present invention, "linseed oil" means the oil obtained by pressing the ripe seeds of the flax plant (*Linum usitatissimum L.*)*,* which have usually been previously dried. Linseed oil is made up mainly of triglycerides and the typical distribution of the fatty acids making it up is generally the following: about 10% saturated fatty acids, about 23% monounsaturated fatty acids, about 67% polyunsaturated fatty acids. Among the polyunsaturated fatty acids, linseed oil is rich mainly in linoleic acid (omega-6) and α-linolenic acid (omega-3), in a ratio of about 1:4. Linseed oil is used in the human diet since the high content of polyunsaturated fatty acids makes it beneficial for the health of the heart and skin and for the immune system. Some studies have also demonstrated an anti-inflammatory action capable of combating pathologies such as rheumatoid arthritis.

In the context of the present invention, "hempseed oil" means a product extracted from the fruits of *Cannabis sativa L.*, generally by means of cold pressing.

Cold-pressed hempseed oil contains about 10% saturated fatty acids and about 90% unsaturated fatty acids, in particular, about 50-60% are omega-6 and about 20% are omega-3.

Hempseed oil is therefore the vegetable oil with the highest content of unsaturated fatty acids. Hempseed oil further has a high content of vitamin E (whose antioxidant action is known), carotene (precursor of vitamin A), phytosterols, phospholipids and minerals, including calcium, magnesium, sulphur, potassium and phosphorus, as well as modest amounts of iron and zinc.

Hempseed oil is used in both the food industry and cosmetic industry (hydrating and anti-aging action) and also has application as a biofuel. Some studies have demonstrated a beneficial action on human health, since it seems to help to prevent heart diseases and to contribute to reducing cholesterol, as well as to combat disorders of the osteoarticular and muscle systems (e.g. arthrosis, arthritis, muscle and joint pains) and autoimmune diseases (rheumatoid arthritis and Crohn's disease).

A first aspect of the present invention makes reference to a mixture of linseed oil and hempseed oil characterised by an omega-6:omega-3 fatty acids ratio (ω6:ω3) comprised between 1:4 and 3:1,consisting of linseed oil and hempseed oil in a volume/volume ratio comprised between 60:40 and 95:5, preferably between 65:35 and 85:15.

The ratio between linseed oil and hempseed oil is preferably comprised between 70:30 and 85:15, more preferably between 70:30 and 80:20, even more preferably between 60:40 and 95:5, even more preferably between 65:35 and 85:15, and even more preferably it is about 75:25. The mixture of oils is used as a solvent for molecules/substances/compounds characterized by low or reduced bioavailability, preferably lipophilic molecules/substances/compounds, more preferably molecules/substances/compounds as described in greater detail below.

A second aspect of the present invention relates to a composition comprising the mixture of linseed oil and hempseed oil as described above.

The composition according to the invention comprises the mixture of linseed oil and hempseed oil in a volume/volume ratio comprised between 60:40 and 95:5, preferably between 65:35 and 85:15.

According to a further preferred embodiment, the composition comprises the mixture of linseed oil and hempseed oil in a ratio comprised between 70:30 and 85:15; more preferably, the composition comprises the mixture of linseed oil and hempseed oil in a ratio comprised between 70:30 and 80:20.

In a particularly preferred embodiment of the invention, the ratio between linseed oil and hempseed oil is 75:25.

In the present text, where reference is made to the ratio between linseed oil and hempseed oil, it is understood that said ratio is expressed as a volume/volume ratio; for example, a volume/volume ratio comprised between 60:40 means that out of a volume of 100ml, 60ml are linseed oil and 40ml are hempseed oil. Analogous reasoning in reference to different ratios.

In any case, in this context, the ratio between linseed oil and hempseed oil can be modified, within the range envisaged by the present invention, in particular in order to optimize the effectiveness of the composition, for example depending on the one or more further substances that can be present in the composition itself, as described below.

In fact, the authors of the present invention have surprisingly found that the mixture consisting of linseed oil and hempseed oil and the composition comprising said mixture of oils in the ratios specified above, are characterized by a ω6/ω3 fatty acid ratio comprised from 1:4 to 3:1, i.e. a ω6/ω3 fatty acid ratio that is defined as balanced from a nutritional viewpoint. In other words, the composition as such represents an important nutritional supplement, as it contributes fatty acids while respecting the body's physiological limits, thus without generating any oxidative stress. From this point of view, the composition of the present invention may also be considered a food or edible composition. According to a preferred aspect of the present invention, the linseed oil and/or the hempseed oil are unrefined oils.

In this regard, the absence of refinement makes it possible to preserve the content of omega-6 and omega-3 fatty acids characteristic of the natural oil, without the alterations and imbalances in the ratios between these fatty acids which were spoken of earlier.

The linseed oil and/or the hempseed oil are preferably obtained by cold pressing.

Advantageously, the mixture of oils and the compositions of the present invention are characterized by the high plasma absorption thereof, i.e. it is greater than the absorption of known lipidic compositions presently used. This capacity of being easily absorbed at the intestinal site and entering the bloodstream enables the composition of linseed oil and hempseed oil to represent an effective system also for the entry into the plasma of other substances with low or reduced bioavailability. That is, they represent a carrier, a system for delivering substances characterized by low or reduced bioavailability in an optimal manner.

In fact, many of the substances that are useful or in any case beneficial for the human body exhibit low bioavailability, often due to poor intestinal absorption.

Therefore, the mixture and the composition of the invention are particularly useful for the purpose of facilitating the intestinal absorption of substances (molecules/compounds) commonly used in the nutraceutical field, preferably natural extracts of vegetable and/or animal origin with low bioavailability, which may also be more or less lipophilic.

According to a preferred embodiment of the invention, the substances commonly used in nutraceuticals and scarcely bioavailable due to their reduced intestinal absorption are selected from the group consisting of: vitamins, polyphenols, flavonoids, isoflavonoids, bioflavonoids, phytoestrogens, carotenoids, dry vegetable extracts in general, lactic ferments, melatonin, coenzyme Q10 and combinations thereof.

According to a further preferred embodiment of the invention, the scarcely bioavailable substances are also molecules with a particularly rapid metabolism, and/or molecules having low water solubility, i.e. lipophilic substances/molecules.

Therefore, according to a preferred embodiment of the invention, the mixture and/or the composition comprising the mixture of linseed oil and hempseed oil in the ratios specified above further comprises one or more beneficial substances with reduced bioavailability as described above. Said beneficial substances are preferably selected from the group consisting of: vitamins, polyphenols, flavonoids, isoflavonoids, bioflavonoids, phytoestrogens, carotenoids, dry vegetable extracts in general, lactic ferments, melatonin, coenzyme Q10, lipophilic substances, molecules with a particularly rapid metabolism and combinations thereof. The amount of melatonin is preferably comprised between 0.5mg and 1 mg, preferably as a daily dose.

According to a preferred embodiment, the vitamins are selected from the group consisting of: vitamin A, vitamin C, vitamin D, vitamin E, vitamin K and combinations thereof.

The lipophilic vitamin D3, also known as cholecalciferol, is particularly preferred.

The amount of vitamin, preferably vitamin D3, is preferably comprised between 5µg and 7.5µg as a daily dose. A smaller dose can be taken twice a day.

According to a further preferred embodiment, the polyphenols are selected from the group consisting of: anthocyanins, curcumin, resveratrol, silimarin and combinations thereof.

According to a further preferred embodiment, the phytoestrogens are preferably isoflavones, more preferably selected from the group consisting of: genistein, daidzin, glycitein, cranberry and combinations thereof. According to a further preferred embodiment, the carotenoids are selected from the group consisting of: astaxanthin, beta-carotene, crocetin, lycopene, lutein, zeaxanthin and combinations thereof.

According to a further preferred embodiment, the preferred flavonoid is quercetin. The amount of quercetin is preferably comprised between 300mg and 1000mg per day. The minimum dose can be taken twice a day.

According to a particularly preferred embodiment, the mixture and/or composition comprising the mixture of linseed oil and hempseed oil in the above-described ratios further comprises at least one ingredient, preferably all the ingredients, selected from the group consisting of: vitamin D, melatonin, quercetin and combinations thereof.

The mixture and/or the composition of the invention is prepared in a conventional manner, by simply mixing the linseed oil with the hempseed oil, in the volume/volume ratio desired for each specific case.

If the composition also comprises one or more further substances, in one embodiment said one or more further substances can be added to the previously prepared composition of the two oils.

Alternatively, the one or more further substances can be added to one of the two oils (linseed oil or hempseed oil) before it is mixed with the other oil to form the composition.

In the case of preparations with several ingredients, the latter may also be dissolved in only one of the two oils separately and mixed together only at the end.

According to a further embodiment of the invention, the mixture of oils in the above-described ratios and/or the composition comprising the two oils and optionally the further ingredients as described above in detail, are subjected to at least one, preferably 1 to 10, more preferably 2 to 8, and more preferably 3 cold mixing cycles.

In the context of the present invention, cold mixing means keeping the preparation under ice during the mixing cycles, so that the internal temperature of the preparation is maintained constant (generally less than 10°C) and is not affected by any increase in temperature caused by the emulsifier during stirring. Furthermore, the mixing instrument is kept in a cold environment (generally at around 4°C) for about 24 hours prior to preparation, i.e. the mixing instrument is pre-cooled.

The cold mixing is preferably carried out in rotation for about 1-10 seconds, preferably 3-5 seconds. Every mixing cycle is preferably followed by a phase of rest of the mixture, preferably under ice.

The mixing is preferably carried out in rotation, preferably for about 1-10 seconds, more preferably 3-5 seconds.

The rotation is preferably carried out at 8000-16000 rpm (revolutions per minute), more preferably at 9000-11000 rpm, even more preferably at 10000 rpm.

The rotation is preferably carried out by means of an emulsifier.

This procedure makes it possible to nanoemulsify the composition of the invention.

The formulation of the composition of the invention as a nanoemulsion, which is obtained with this process, is particularly advantageous, since it enables one to have a complete mutual dispersion of the two oils which would otherwise tend to become stratified in phases according to their density. That is, the nanoemulsfied composition remains perfectly homogeneous over time. Furthermore, the addable substances are better dispersed.

In order to be able to prepare the nanoemulsion as described above, it is advisable to use a mixer for the preparation of emulsions that is calibrated according to the final volume to be prepared (in the illustrated examples, use was made of an instrument for small volumes, preferably 1 millilitre at most).

Therefore, a further aspect of the present invention relates to a nanoemulsion comprising the combination of linseed oil and hempseed oil in the above-described ratios with and/or without one or more of the further substances/ingredients previously described in detail.

Finally, a further aspect of the present invention is that said nanoemulsion comprising the combination of linseed oil and hempseed oil in the above-described ratios with or without one or more of the further substances/ingredients previously described in detail can be used in the cosmetics industry and/or in any case for improving individual well-being.

In fact, by virtue of the particular ratio between the linseed oil and hempseed oil present in the composition, the composition itself provides a balanced input of omega-6 and omega-3 fatty acids, which is thus beneficial for the human body. This balanced input, in particular, corresponds to physiological conditions and therefore will not provoke oxidative stress at the cellular level.

Furthermore, the composition of the invention is easily absorbed at the intestinal site and also in brief periods of time. In fact, the absorption may be found in percentages even greater than 50% of the amount administered even only 1 hour after administration (situation typical of intake on an empty stomach). The ease of intestinal absorption of the composition of linseed and hempseed oil enables the composition to carry into the bloodstream further substances beneficial for the body that are added to the composition itself, for example the previously described substances having low bioavailability due to poor absorption, namely, vitamins, polyphenols, flavonoids, isoflavonoids, bioflavonoids, phytoestrogens, carotenoids, dry vegetable extracts, lactic ferments, melatonin, coenzyme Q10, lipophilic substances, molecules with a particularly rapid metabolism and combinations thereof.

Therefore, a further aspect of the present invention relates to the use of the composition and/or nanoemulsion as described above as a medicament, in particular for the prevention and/or the cure/therapeutic treatment and/or during the follow-up phase or following the occurrence (in order to reduce the risk of relapses) of a pathology selected from the group consisting of: degenerative diseases in the neurological, ophthalmic, gynecological, cardiovascular, gastrointestinal, osteoarticular and muscular fields and malabsorption syndrome. The pathologies are preferably selected from the group consisting of: glaucoma, retinopathies and maculopathy, chronic stroke, neurodegenerative diseases, pre-menstrual syndrome and menopause, polycystic ovary syndrome, coronary heart disease, hypertension, cardiac failure, as a cardiac adjuvant for long-term pharmacological treatments, ulcer, hyperacidity and gastro-esophageal reflux, chronic inflammatory diseases (rectocolitis and Chron's disease), rheumatic diseases, and for post-traumatic and rehabilitation treatments.

In general, the composition and/or the nanoemulsion as described above are used to increase the absorption and/or the metabolism and/or the bioavailability, preferably at the intestinal site, of molecules, preferably of molecules with low bioavailability, more preferably drugs, food supplements and other orally administered compounds. Said molecules are preferably selected from the group consisting of: vitamins, preferably selected from the group consisting of: vitamin A, vitamin C, vitamin D, vitamin E, vitamin K and combinations thereof; polyphenols, preferably selected from the group consisting of: anthocyanins, curcumin, resveratrol, silimarin and combinations thereof; flavonoids, preferably quercetin, preferably isoflavones, more preferably selected from the group consisting of: genistein, daidzin, glycitein, cranberry and combinations thereof; bioflavonoids, phytoestrogens, carotenoids, preferably selected from the group consisting of: astaxanthin, beta-carotene, crocetin, lycopene, lutein, zeaxanthin and combinations thereof, dry vegetable extracts in general, lactic ferments, melatonin, coenzyme Q10, lipophilic substances, molecules with a particularly rapid metabolism and combinations thereof.

### EXAMPLE

### Cell culture

In the experiments use was made of cells of the CaCo-2 line, that is, a human epithelial cell line of colon cancer commonly used and accepted as a model for studying the absorption, metabolism and bioavailability, at the intestinal site, of drugs, food supplements and other orally administered compounds.

The cells were kept in a complete culture medium - Dulbecco's Modified Eagle's Medium/Nutrient F-12 Ham (DMEM-F12, Sigma-Aldrich, Milan Italy) - containing 10% fetal bovine serum (FBS, Sigma-Aldrich, Milan Italy), 2mM of L-glutamine (Sigma-Aldrich, Milan Italy) and 1% penicillin-streptomycin (Sigma-Aldrich, Milan Italy) and placed in an incubator at 37°C with 5% CO₂.

The cells were used under different experimental conditions:
- 1×10⁴ cells were seeded in 96-well plates in order to test cell viability by means of an MTT assay;
- 1×10⁴ cells were seeded in 96-well plates in order to assess lipid peroxidation;
- for the absorption studies, 2×10⁴ cells were seeded in 6.5 mm Transwells with a 0.4 µm polycarbonate insert (Sigma-Aldrich, Milan Italy) for 24-well plates.

Before the treatments were carried out (except in the case of the absorption studies), the cells were washed and incubated so as to synchronise the sample, in DMEM without phenol red and FBS, at 2mM of L-glutamine (Sigma-Aldrich, Milan Italy) and 1% penicillin-streptomycin (Sigma-Aldrich, Milan Italy), for about 8h in an incubator at 37°C with 5% CO₂.

The cells seeded in the Transwells were kept in the complete medium for 21 days before the treatment and the medium was changed every other day, first in the basolateral chamber and then the apical one.

### Experimental protocol

The CaCo-2 cells were stimulated in DMEM blank 0.5% FBS for 1h and 3h in an incubator at 37°C with the compositions comprising linseed oil (L) and hempseed oil (C) in different ratios (from 50%L+50%C to 95%L+5%C); for comparative purposes, the cells were also stimulated with the individual components at 100% (100%L and 100%C).

The range that showed to be most efficient (from 70-30 to 85-15, as defined in the figures) was subsequently tested in association with several molecules typically present in the main food supplements for human use. This test was carried out to demonstrate the safety, tolerability and range of effectiveness in association with: 1) vitamin D3 (100nM), 2) melatonin (50µM); and 3) quercetin (50µM) after 1h and after 3h of treatment.

The data were compared with those of preparations comprising vitamin D3 (100nM), melatonin (50µM) or quercetin (50µM) in 100% ethanol.

### MTT test

In order to determine cell viability over time following different treatments, use was made of the commercial kit "In Vitro Toxicology Assay Kit" (Sigma-Aldrich, Milan Italy), which uses MTT (3-[4,5-dimethylthiazol-2-y1]-2,5-diphenyltetrazoliumbromide) to determine the cytotoxicity of the treatments.

After stimulation carried out as described above, the cells were incubated with 1% of MTT prepared in blank serum-free DMEM for 2-4h at 37°C in an incubator. Once the incubation period had elapsed, the medium was removed and the formazan crystals forming on the bottom of the well were solubilized with an equal volume, compared to the original incubation medium, of the "MTT Solubilization Solution" included in the kit.

Viability was determined by reading the supernatant with a spectrophotometer (VICTORX3 Multilabel Plate Reader) at a wavelength of 570 nm with correction at 690 nm and calculated as a percentage relative to the untreated control cells (100% viability).

### Lipid peroxidation

In order to determine any lipid peroxidation present after the different treatments, use was made of the "Lipid Peroxidation (MDA) Assay Kit" (Sigma-Aldrich, Milan Italy).

Peroxidation is assessed through the reaction between malonyldialdehyde (MDA) and thiobarbituric acid (TBA), which leads to the formation of a colorimetric product proportional to the amount of MDA present in the sample. At the end of the stimulations, the samples were homogenized in MDA Lysis Buffer and centrifuged; a TBA solution was subsequently added to the pellets, which were incubated at 95 °C for 1 hour. The absorbance of the samples was measured with a spectrophotometer (VICTORX3 Multilabel Plate Reader) at a wavelength of 532 nm and peroxidation was calculated as a percentage relative to an untreated sample (control), normalized relative to the internal positive control (200 µM H₂O₂).

### Intestinal absorption

After 21 days of maintenance, the cells were treated for 1h (simulating an administration on an empty stomach) and for 3h (simulating an administration after a meal) on the apical side at pH 6.5, in order to verify the passage of the compounds into the basolateral environment at pH 7.4. This difference in pH simulates the actual passage from the intestinal lumen to the blood in the human body after oral intake.

Both the composition consisting of the vegetable oils alone and the composition supplemented with vitamin D3 (100 nM), melatonin (50µM) or quercetin (50µM) were added to the apical chamber. 30 min before the end of the treatments, 100 µl of cytochrome C (Sigma-Aldrich, Milan Italy) were added as a coloured marker in the apical zone; at the end of incubation, the volume in the apical chamber and basal chamber were measured, and the content of the basal chamber was read with a spectrophotometer (VICTORX3 Multilabel Plate Reader) at the length of 550 nm. The percentage of absorption was calculated based on the intensity measured in the basal chamber relative to the untreated control sample, normalized on the observed difference in volume and relative to the values obtained by the sample treated with propranolol (positive control of high permeability).

### Preparation of the solutions.

In the compositions examined, linseed oil and hempseed oil are contained in proportions that range from 95% to 50% of linseed oil and from 5% to 50% of hempseed oil. The composition supplemented with other ingredients is evaluated each time on the basis of the observed absorption values.

The composition comprising solely the combination of oils or those further comprising other substances (vitamin D, quercetin, etc..) are also prepared following a further optimized protocol that provides for 3 cold mixing cycles at about 10000 rpm lasting about 3-5 seconds, with rest intervals of 20 sec under ice between them, using a small-volume mixer (max 1ml) for preparing emulsions.

### Statistical analysis

The results are expressed as the mean ± standard deviation (SD) of at least 4 independent experiments for each experimental protocol. The different treatment groups were compared by one-way ANOVA with Bonferroni correction, or using a Mann-Whitney U test; the results were converted into graphs by means of Graph Pad Prism 5 (Graph Pad Software, La Jolla, CA, USA). Values of p<0.05 were considered statistically significant.

### Dose-response study of cell viability

In order to study gastrointestinal absorption, it is necessary to consider the possible cytotoxicity of the composition. The composition was thus tested first of all in terms of its effect on cell viability as an indicator of a possible beneficial effect at a systemic level. As reported in Figs. 1A and 1B, which show the cell viability measured 1 hour (A) and 3 hours (B) after the various stimulations, a greater compliance was observed for the mixtures of linseed oil (L) and hempseed oil (C) compared to the single pure products (100%L and 100%C) (p<0.05). Furthermore, it may be observed that there is no substantial difference in the effect of the pure oils (100%L and 100%C) 1 hour after treatment, whereas after 3 hours the effect on cell viability shows an increase, with a greater effectiveness of hempseed oil (p<0.05).

As regards the compositions with different percentages of linseed oil and hempseed oil, it was possible to observe that the effect on cell viability is both time-dependent (all the compositions at different percentages showed a greater effect after 3 hours of treatment) and concentration-dependent: this demonstrates that the balance between omega-3 and omega-6 is fundamental for a better functionality of the composition. Each oil was added directly to the medium in order to obtain the final concentrations, which ranged from 5% to 100%. From both graphs (Figs. 1A and 1B) it may be observed that the most effective compositions in terms of viability are the ones comprising linseed oil in a percentage comprised between 70% and 85% and hempseed oil in a percentage comprised between 15% and 30%. The results shown are expressed as the mean ± SD (%), normalized relative to the untreated sample (control) of 4 independent experiments, each conducted in triplicate.

### Dose-response study of lipid peroxidation

The use of vegetable oils can generate products of oxidation which will potentially accumulate with harmful results; it is therefore important to assess the possible lipid peroxidation in the cell membrane. Lipid peroxidation (understood as a degradation of membrane lipids) is a marker of oxidative damage leading to the formation of high levels of free radicals or reactive species of oxygen which are a cause of cell damage. In order to examine lipid peroxidation, an assay was used which evaluates the production of malondialdehyde induced by linseed and hempseed oils, either pure or mixed together, compared to an untreated control sample. The data obtained from these experiments made it possible to rule out a state of oxidation of the cells and thus demonstrate the safety and tolerability of the components used. Each oil was diluted directly in the medium so as to obtain a final concentration ranging from 5% to 100%. As may be observed from Fig.2A, the pure linseed oil (100%L) showed a time-dependent increase in peroxidation (p<0.05) compared to the pure hempseed oil (100%C) and the mixtures of the two oils; conversely, the pure hempseed oil (100%C) showed a lower degree of lipid peroxidation than the pure linseed oil (100%L) (p<0.05) in both observation time periods and it was significant only in some cases compared to the mixtures of the two oils. The levels of lipid peroxidation in any case remained within safe levels (lower than the levels of peroxidation induced by H₂O₂, which are around 50%). As regards, on the other hand, the peroxidation induced by the mixtures of oils, it may be observed that there are no substantial differences in terms of percentage composition of the oils and, even though the percentage of peroxidation increases over time (Figs. 2A and 2B), it always remains within safe limits. The results shown are expressed as the mean ± SD (%), normalized relative to the untreated sample (control) of 4 independent experiments, each conducted in triplicate.

### Study of intestinal absorption over time

The aim of this study is to evaluate the ability of cells to absorb, on the luminal side and basolateral side, both the pure linseed and hempseed oils, and the mixtures of oils in various percentages. Absorption studied in vitro is able to reflect absorption at the intestinal site and the ability of the oils (and of the substances carried by them) to reach the bloodstream: at an experimental level, the absorption conditions were recreated through the different pHs of the two chambers (corresponding to the intestinal pH and blood pH). Each oil was diluted directly in the medium so as to obtain a final concentration ranging from 5% to 100%. In these experiments, the absorption capacity at 1 hour and at 3 hours was analysed (Figs. 3A and 3B respectively) in order to simulate the different ways in which the most common supplements are taken. When taken on an empty stomach, absorption occurs in about 1 hour after intake; when taken on a full stomach, absorption occurs in about 3 hours. The pure linseed and hempseed oils do not seem to modulate their absorption over time, though they reach slightly higher levels after 3 hours.

As regards the mixtures of oils at different concentrations, the 70-30, 75-25, 80-20 and 85-15 mixtures (as defined in Figures 1-3) show to be the most efficient (p<0.05), compared both to the controls and the compositions of pure oils, as well as compared to the compositions comprising mixtures of oils at the remaining concentrations: these mixtures reach 50% absorption both 1 hour and 3 hours after treatment. These data provide an indication as to the percentage ranges of the linseed oil and hempseed oil composition that enable an effective intestinal absorption of the substances carried by the composition itself following oral administration both on a full stomach and on an empty stomach. The results shown are expressed as the mean ± SD (%), normalized relative to the untreated sample (control) of 4 independent experiments, each conducted in triplicate.

### Study of cell viability (with a composition of oils supplemented with vitamin D3, melatonin or quercetin)

In order to rule out any cytotoxicity of the composition of oils in the event that is associated with other molecules, cell viability tests were conducted to determine the effectiveness of the composition of oils in association with different molecules. In particular, the 70-30, 75-25, 80-20, 85-15 oil mixtures (as defined in figures 1-3) supplemented with vitamin D3, or melatonin, or quercetin were tested. The treatments of the cells were maintained for 1 hour and 3 hours and compared with the effect on cells of compositions comprising vitamin D3, melatonin or quercetin in ethanol. As may be observed from Figs. 4A and 4B, cell viability showed to be better for the combinations of vitamin D3, quercetin and melatonin with the oil compositions, compared to the combinations of vitamin D3, quercetin and melatonin with ethanol; furthermore, the effect is time-dependent.

In particular, as regards the compositions comprising vitamin D3, it may be observed that the one that showed to be particularly effective in terms of cell viability both at 1 hour and at 3 hours was the one comprising linseed oil and hempseed oil in percentages of 80% and 20%, respectively. This concentration was statistically significant compared both to the composition in ethanol and the other oil-based compositions (p<0.05).

As regards the compositions comprising quercetin, they too act in a time-dependent manner; in this case as well, the composition that showed to be most effective in terms of cell viability was the one comprising linseed oil and hempseed oil in percentages of 80% and 20%, respectively (with p<0.05).

Finally, the compositions comprising melatonin were the ones that gave the best effects in terms of cell viability after 1 hour of treatment; the most effective composition, compared both to the composition in ethanol and to the other oil-based compositions (p<0.05), was the one comprising linseed oil and hempseed oil in percentages of 85% and 15%, respectively. The viability profile remained similar also 3 hours after the treatment. These studies showed the ability of the compositions comprising linseed oil and hempseed oil to enable substances added to them (vitamin D3, quercetin, melatonin) to express their beneficial biological effects. The compositions that offered the best results in this respect were the 80-20 and 85-15 ones (as defined in Figures 1-3); therefore, they will be the focus of the next study. The results shown are expressed as the mean ± SD (%), normalized relative to the untreated sample (control) of 4 independent experiments, each conducted in triplicate.

### Study of the intestinal absorption of vitamin D3, melatonin or quercetin added to the compositions of linseed oil and hempseed oil

After having demonstrated the effectiveness and ruled out the cytotoxicity of the compositions comprising mixtures of linseed oil and hempseed oil in various percentages, supplemented with vitamin D3, melatonin or quercetin, it is important to assess the absorption *in vitro* of such compositions, from which one may assume the plasma absorption of the same compositions in humans after oral administration. The conditions of intestinal absorption were recreated *in vitro* through the different pH levels in the chambers, which correspond to intestinal pH and blood pH. In these experiments, the absorption capacity at 1 hour and at 3 hours (Figs. 4A and 4B, respectively) was analysed to simulate the different ways in which the most common supplements are taken. When taken on an empty stomach, absorption occurs in about 1 hour after intake; when taken on a full stomach, absorption occurs in about 3 hours.

The compositions comprising mixtures of oils and vitamin D3, or melatonin, or quercetin showed a higher degree of absorption than the compositions comprising ethanol and the same substances. Furthermore, absorption showed to be time-dependent, an indicator that, irrespective of the way they are taken, vitamin D3, melatonin or quercetin enter the bloodstream correctly. As shown in Fig. 5A, the three molecules concerned (vitamin D3, melatonin, quercetin) are all absorbed at a level of more than 50%, which is maintained also 3 hours after treatment (Fig. 5B). These data demonstrate the importance of the composition comprising the vegetable oils in ensuring the intestinal absorption of the molecules over time, consistently with the physiological bowel emptying. The results shown are expressed as the mean ± SD (%), normalized relative to the untreated sample (control) of 4 independent experiments, each conducted in triplicate.

In the light of the foregoing, it has been ascertained in practice that the composition according to the present invention fully achieves the preestablished task, as it exhibits a high capacity to be absorbed at the intestinal site, thereby reaching the bloodstream. This same capacity enables the composition to act as a carrier of many substances commonly used as food supplements or components of special-purpose foods having low bioavailability, as it increases the intestinal absorption thereof and consequently the bioavailability thereof in plasma.

Furthermore, the composition according to the present invention possesses a balanced content of omega-6 and omega-3 fatty acids, thanks to which the composition is safe and nutritionally optimal for human dietary use.

## Claims

1. A mixture consisting of linseed oil and hempseed oil **characterized by** an omega-6:omega-3 fatty acids ratio (ω6:ω3) comprised between 1:4 and 3:1, wherein the volume/volume ratio between linseed oil and hempseed oil is comprised between 60:40 and 95:5, preferably is comprised between 65:35 and 85:15.

2. The mixture according to claim 1, wherein the ratio between linseed oil and hempseed oil is comprised between 70:30 and 85:15, preferably is comprised between 70:30 and 80:20, and more preferably it is 75:25.

3. Composition comprising the mixture according to claim 1 or claim 2 and at least one molecule, compound, substance **characterized by**: 1) low bioavailability; and/or 2) rapid metabolism; and/or 3) low water solubility, said molecule, compound, substance being preferably selected from the group consisting of: vitamins, polyphenols, flavonoids, isoflavonoids, bioflavonoids, phytoestrogens, carotenoids, vegetable extracts, lactic ferments, melatonin, coenzyme Q10, lipophilic substances, molecules with a particularly rapid metabolism, and combinations thereof.

4. The composition according to claim 3 wherein: 1) the vitamins are selected from the group consisting of: vitamin A, vitamin C, vitamin D, preferably vitamin D3, vitamin E, vitamin K and combinations thereof; 2) the polyphenols are selected from the group consisting of: anthocyanins, curcumin, resveratrol, silimarin, and combinations thereof; 3) the phytoestrogens are preferably isoflavons, more preferably selected from the group consisting of: genistein, daidzin, glycitein, cranberry and combinations thereof; 4) the carotenoids are selected from the group consisting of: astaxanthin, beta-carotene, crocetin, lycopene, lutein, zeaxanthin and combinations thereof; 5) the preferred flavonoid is quercetin.

5. Process for nanoemulsifying the mixture according to claim 1 or claim 2 or the composition according to claim 3 or claim 4 comprising at least one mixing step, preferably 3-10 mixing steps, at a temperature lower than 10°C, wherein said mixing step is preferably carried out in rotation for about 1-10 seconds, preferably 3-5 seconds, wherein said rotation is preferably carried out at 8000-16000 rpm (revolutions per minute), preferably at 9000-11000 rpm, more preferably at 10000 rpm, said rotation being preferably carried out by means of an emulsifier.

6. The mixture according to claim 1 or claim 2 or the composition according to claim 3 or claim 4, formulated as nanoemulsion.

7. The mixture according to claim 1 or claim 2 or the composition according to claim 3 or claim 4 or the nanoemulsion according to claim 6, formulated as lozenges, tablets, pills, granules, preferably for oral administration, or as a cream, gel, lotion, ointment, spray, tissue, preferably for topical applications, preferably for sports and/or cosmetic and/or nutraceutical applications.

8. The mixture according to claim 1 or claim 2 or of the composition according to claim 3 or claim 4 or of the nanoemulsion according to claim 6 for use to increase the absorption and/or the metabolism and/or the bioavailability, preferably at the intestinal site, of molecules that are drugs.

9. The mixture according to claim 1 or claim 2 or the composition according to claim 3 or claim 4 or the nanoemulsion according to claim 6 for use as a medicament.

10. The mixture according to claim 1 or claim 2 or the composition according to claim 3 or claim 4 or the nanoemulsion according to claim 6 for use in the prevention and/or in the therapeutic treatment and/or in the follow-up of a pathology selected from the group consisting of degenerative diseases in the neurological, ophthalmic, gynecological, cardiovascular, gastrointestinal, osteoarticular, muscular fields and malabsorption syndrome, preferably said pathology being selected from the group consisting of glaucoma, retinopathies and maculopathy, chronic stroke, neurodegenerative diseases, pre-menstrual syndrome and menopause, polycystic ovary syndrome, coronary heart disease, hypertension, cardiac failure, cardiac adjuvant for long-term pharmacological treatments, ulcer, hyperacidity and gastro-esophageal reflux, chronic inflammatory diseases (rectocolitis and Chron), rheumatic diseases, post-traumatic and rehabilitation treatments.

11. Use of mixture according to claim 1 or claim 2 or of the composition according to claim 3 or claim 4 or of the nanoemulsion according to claim 6 as a cosmetic.

## Patentansprüche

1. Mischung, bestehend aus Leinöl und Hanfsamenöl, **gekennzeichnet durch** ein Omega-6:Omega-3-Fettsäuren-Verhältnis (ω6:ω3) zwischen 1:4 und 3:1, wobei das Volumen/Volumen-Verhältnis zwischen Leinöl und Hanfsamenöl zwischen 60:40 und 95:5, vorzugsweise zwischen 65:35 und 85:15 liegt.

2. Mischung nach Anspruch 1, wobei das Verhältnis zwischen Leinöl und Hanfsamenöl zwischen 70:30 und 85:15, vorzugsweise zwischen 70:30 und 80:20 und besonders bevorzugt bei 75:25 liegt.

3. Zusammensetzung, umfassend die Mischung nach Anspruch 1 oder Anspruch 2 und mindestens ein Molekül, eine Verbindung, eine Substanz, **gekennzeichnet durch**: 1) geringe Bioverfügbarkeit; und/oder 2) schnellen Stoffwechsel; und/oder 3) geringe Wasserlöslichkeit, wobei das Molekül, die Verbindung, die Substanz vorzugsweise aus der Gruppe ausgewählt ist, bestehend aus: Vitaminen, Polyphenolen, Flavonoiden, Isoflavonoiden, Bioflavonoiden, Phytoöstrogenen, Carotinoiden, pflanzlichen Extrakten, Milchfermenten, Melatonin, Coenzym Q10, lipophilen Substanzen, Molekülen mit einem besonders schnellen Stoffwechsel und Kombinationen davon.

4. Zusammensetzung nach Anspruch 3, wobei: 1) die Vitamine aus der Gruppe ausgewählt sind, bestehend aus: Vitamin A, Vitamin C, Vitamin D, vorzugsweise Vitamin D3, Vitamin E, Vitamin K und Kombinationen davon; 2) die Polyphenole sind aus der Gruppe ausgewählt, bestehend aus: Anthocyaninen, Curcumin, Resveratrol, Silimarin und Kombinationen davon; 3) die Phytoöstrogene sind vorzugsweise Isoflavone, bevorzugter ausgewählt aus der Gruppe bestehend aus: Genistein, Daidzin, Glycitein, Cranberry und Kombinationen davon; 4) die Carotinoide sind aus der Gruppe ausgewählt, bestehend aus: Astaxanthin, Beta-Carotin, Crocetin, Lycopin, Lutein, Zeaxanthin und Kombinationen davon; 5) das bevorzugte Flavonoid ist Quercetin.

5. Verfahren zum Nanoemulgieren der Mischung nach Anspruch 1 oder Anspruch 2 oder der Zusammensetzung nach Anspruch 3 oder Anspruch 4, umfassend mindestens einen Mischschritt, vorzugsweise 3-10 Mischschritte, bei einer Temperatur von weniger als 10 °C, wobei der Mischschritt vorzugsweise in Rotation für etwa 1-10 Sekunden, vorzugsweise 3-5 Sekunden, durchgeführt wird, wobei die Rotation vorzugsweise bei 8000-16000 U/min (Umdrehungen pro Minute), vorzugsweise bei 9000-11000 U/min, bevorzugter bei 10000 U/min, durchgeführt wird, wobei die Rotation vorzugsweise mittels eines Emulgators durchgeführt wird.

6. Mischung nach Anspruch 1 oder Anspruch 2 oder Zusammensetzung nach Anspruch 3 oder Anspruch 4, formuliert als Nanoemulsion.

7. Mischung nach Anspruch 1 oder Anspruch 2 oder Zusammensetzung nach Anspruch 3 oder Anspruch 4 oder Nanoemulsion nach Anspruch 6, formuliert als Lutschtabletten, Tabletten, Pillen, Granulate, vorzugsweise zur oralen Verabreichung, oder als Creme, Gel, Lotion, Salbe, Spray, Gewebe, vorzugsweise für topische Anwendungen, vorzugsweise für sportliche und/oder kosmetische und/oder nutrazeutische Anwendungen.

8. Mischung nach Anspruch 1 oder Anspruch 2 oder der Zusammensetzung nach Anspruch 3 oder Anspruch 4 oder der Nanoemulsion nach Anspruch 6 zur Verwendung zur Erhöhung der Resorption und/oder des Stoffwechsels und/oder der Bioverfügbarkeit, vorzugsweise an der Darmstelle, von Molekülen, die Arzneimittel sind.

9. Mischung nach Anspruch 1 oder Anspruch 2 oder Zusammensetzung nach Anspruch 3 oder Anspruch 4 oder Nanoemulsion nach Anspruch 6 zur Verwendung als Medikament.

10. Mischung nach Anspruch 1 oder Anspruch 2 oder Zusammensetzung nach Anspruch 3 oder Anspruch 4 oder Nanoemulsion nach Anspruch 6 zur Verwendung bei der Prävention und/oder bei der therapeutischen Behandlung und/oder bei der Folgeuntersuchung einer Pathologie, ausgewählt aus der Gruppe bestehend aus degenerativen Erkrankungen im neurologischen, ophthalmologischen, gynäkologischen, kardiovaskulären, gastrointestinalen, osteoartikulären, muskulären Bereich und Malabsorptionssyndrom, wobei die Pathologie vorzugsweise aus der Gruppe bestehend aus Glaukom, Retinopathien und Makulopathie, chronischem Schlaganfall, neurodegenerativen Erkrankungen, prämenstruellem Syndrom und Menopause, polyzystischem Ovarsyndrom, koronarer Herzkrankheit, Hypertonie, Herzinsuffizienz, kardialem Adjuvans für langfristige pharmakologische Behandlungen, Geschwüren, Hyperazidität und gastroösophagealem Reflux, chronischen entzündlichen Erkrankungen (Rektokolitis und Chron), rheumatischen Erkrankungen, posttraumatischen und Rehabilitationsbehandlungen ausgewählt ist.

11. Verwendung der Mischung nach Anspruch 1 oder Anspruch 2 oder der Zusammensetzung nach Anspruch 3 oder Anspruch 4 oder der Nanoemulsion nach Anspruch 6 als Kosmetikum.

## Revendications

1. Mélange, constitué d'huile de lin et d'huile de chanvre, **caractérisé par** un rapport entre les acides gras oméga-6 et oméga-3 (ω6 : ω3) compris entre 1:4 et 3:1, dans lequel le rapport volume/volume entre l'huile de lin et l'huile de chanvre est compris entre 60:40 et 95:5, de préférence entre 65:35 et 85:15.

2. Mélange selon la revendication 1, dans lequel le rapport entre l'huile de lin et l'huile de chanvre est compris entre 70:30 et 85:15, de préférence est compris entre 70:30 et 80:20, et plus préférablement est de 75:25.

3. Composition, comprenant le mélange selon la revendication 1 ou la revendication 2 et au moins une molécule, un composé, une substance caractérisé(e) par : 1) une faible biodisponibilité ; et/ou 2) un métabolisme rapide ; et/ou 3) une faible hydrosolubilité, ladite molécule, ledit composé, ladite substance étant de préférence choisi(e) dans le groupe constitué par : les vitamines, les polyphénols, les flavonoïdes, les isoflavonoïdes, les bioflavonoïdes, les phytoœstrogènes, les caroténoïdes, les extraits végétaux, les ferments lactiques, la mélatonine, la coenzyme Q10, les substances lipophiles, les molécules à métabolisme particulièrement rapide, et leurs combinaisons.

4. Composition selon la revendication 3, dans laquelle : 1) les vitamines sont choisies dans le groupe constitué par : la vitamine A, la vitamine C, la vitamine D, de préférence la vitamine D3, la vitamine E, la vitamine K et leurs combinaisons ; 2) les polyphénols sont choisis dans le groupe constitué par : les anthocyanines, la curcumine, le resvératrol, la silymarine et leurs combinaisons ; 3) les phyto-œstrogènes sont de préférence des isoflavones, plus préférablement choisis dans le groupe constitué par : la génistéine, la daidzine, la glycitéine, la canneberge et leurs combinaisons ; 4) les caroténoïdes sont choisis dans le groupe constitué par : l'astaxanthine, le bêta-carotène, la crocétine, le lycopène, la lutéine, la zéaxanthine et leurs combinaisons ; 5) le flavonoïde préféré est la quercétine.

5. Procédé de nanoémulsification du mélange selon la revendication 1 ou la revendication 2 ou de la composition selon la revendication 3 ou la revendication 4, comprenant au moins une étape de mélange, de préférence 3-10 étapes de mélange, à une température inférieure à 10 °C, dans lequel ladite étape de mélange est de préférence effectuée en rotation pendant environ 1-10 secondes, de préférence 3-5 secondes, dans lequel ladite rotation est de préférence effectuée à 8 000-16 000 tr/min (tours par minute), de préférence à 9 000-11 000 tr/min, plus préférablement à 10 000 tr/min, ladite rotation étant de préférence effectuée à l'aide d'un émulsifiant.

6. Mélange selon la revendication 1 ou la revendication 2 ou composition selon la revendication 3 ou la revendication 4, formulé(e) comme une nanoémulsion.

7. Mélange selon la revendication 1 ou la revendication 2 ou composition selon la revendication 3 ou la revendication 4 ou nanoémulsion selon la revendication 6, formulé(e) sous forme de pastilles, comprimés, pilules, granulés, de préférence pour une administration orale, ou sous forme de crème, gel, lotion, pommade, spray, tissu, de préférence pour des applications topiques, de préférence pour des applications sportives et/ou cosmétiques et/ou nutraceutiques.

8. Mélange selon la revendication 1 ou la revendication 2 ou composition selon la revendication 3 ou la revendication 4 ou nanoémulsion selon la revendication 6, pour une utilisation servant à augmenter l'absorption et/ou le métabolisme et/ou la biodisponibilité, de préférence au niveau intestinal, de molécules qui sont des médicaments.

9. Mélange selon la revendication 1 ou la revendication 2 ou composition selon la revendication 3 ou la revendication 4 ou nanoémulsion selon la revendication 6, pour une utilisation comme un médicament.

10. Mélange selon la revendication 1 ou la revendication 2 ou composition selon la revendication 3 ou la revendication 4 ou nanoémulsion selon la revendication 6, pour une utilisation dans la prévention et/ou dans le traitement thérapeutique et/ou dans le suivi d'une pathologie choisie dans le groupe constitué par les maladies dégénératives dans les domaines neurologique, ophtalmique, gynécologique, cardiovasculaire, gastrointestinal, ostéo-articulaire, musculaires et le syndrome de malabsorption, de préférence ladite pathologie étant choisie dans le groupe constitué par le glaucome, les rétinopathies et la maculopathie, l'accident vasculaire cérébral chronique, les maladies neurodégénératives, le syndrome prémenstruel et la ménopause, le syndrome des ovaires polykystiques, les maladies coronariennes, l'hypertension, l'insuffisance cardiaque, l'adjuvant cardiaque pour les traitements pharmacologiques de longue durée, l'ulcère, l'hyperacidité et le reflux gastro-œsophagien, les maladies inflammatoires chroniques (rectocolite et Chron), les maladies rhumatismales, les traitements post-traumatiques et de réadaptation.

11. Utilisation du mélange selon la revendication 1 ou la revendication 2 ou de la composition selon la revendication 3 ou la revendication 4 ou de la nanoémulsion selon la revendication 6, comme un cosmétique.
